# EUROPÄISCHE PATENTSCHRIFT

(11) **EP 1 016 404 B1**
(45) Veröffentlichungstag und Bekanntmachung des Hinweises auf die Patenterteilung: **25.06.2003**
(21) Anmeldenummer: 99890013.8
(22) Anmeldetag: 22.01.1999
(51) Int. Cl.: A61K 31/015, A61K 9/107

(54) **Wässerige Zubereitung von Beta-Carotin**
Aqueous preparation of beta-carotene
Préparation aqueuse de béta-carotène

(30) Priorität: 15.12.1998 AT 209298
(43) Veröffentlichungstag der Anmeldung: 05.07.2000
(73) Patentinhaber: Alvetra Ltd., Valletta (MT)
(72) Erfinder: Frantsits, Werner J., Dr., 1130 Wien (AT)
(74) Vertreter: Beer, Manfred, Dipl.-Ing.

(56) Entgegenhaltungen:
- EP-A- 0 055 817
- EP-A- 0 479 066
- DE-A- 19 609 477
- FR-A- 2 187 291
- DATABASE WPI Week 8307 Derwent Publications Ltd., London, GB; AN 1983-16683k XP002135796 & RO 79 496 A (INTR PROD COSMETICE FARMEC) , 30. Juli 1982 (1982-07-30)

## Beschreibung

Die Erfindung betrifft eine Zubereitung von Beta-Carotin in einem wässerigen Medium zur parenteralen Verabreichung, in Form einer mizellaren Lösung, die Lösungsvermittler enthält. Die Erfindung betrifft weiters ein Verfahren zum Herstellen dieser Zubereitung.

Da unter den Bedingungen der Intensivhaltung von Nutztieren häufig eine die Reproduktionsfähigkeit beeinträchtigende Defizienz an Beta-Carotin vorliegt, wird Beta-Carotin in der Veterinärmedizin seit geraumer Zeit eingesetzt, u.zw. sowohl als Fütterungsarzneimittel, als auch insbesondere in Form von Injektionspräparaten zur schnellen Behebung akuter Mangelerscheinungen.

Die pharmakologische Wirksamkeit von Beta-Carotin hinsichtlich reproduktionsfördernder Eigenschaften, wie Stabilisierung des Gelbkörpers, die Erhöhung des Progesteronspiegels im Plasma, sowie auch allgemein der Aufrechterhaltung der bestehenden Trächtigkeit ist bekannt. Bekannt ist auch, daß die Verabreichung von Beta-Carotin eine Verbesserung der Immunlage des Wurfes herbeiführt.

Injizierbares Carotin findet auch bei der Therapie der Endometriose von Tieren, beispielsweise von Rind, Pferd, sowie Hund Anwendung.

Beta-Carotin ist in Wasser und in gängigen, pharmazeutisch annehmbaren, wässerigen Formulierungen praktisch unlöslich. Beta-Carotin ist aber auch in Ethanol, Cyclohexan und Äther kaum löslich. Ein Problem besteht weiters darin, daß gelöstes Beta-Carotin durch Luftsauerstoff in Gegenwart von Licht und Wärme leicht zersetzt wird.

Die vorgenannten Eigenschaften von Beta-Carotin stellen somit eine galenische Herausforderung dar, da die für ein Arzneimittel geforderte Bioverfügbarkeit und Stabilität nicht bzw. nur ungenüged gewährleistet werden konnte.

Bei Versuchen zur Entwicklung oraler Verabreichungsformen wurde verschiedentlich auf ölige Lösungen (Olivenöl usw.) zurückgegriffen, die entweder als solche oder aber in Form wässeriger Emulsionen gegeben wurden.

Zur parenteralen Verabreichung sind jedoch ölige Lösungen von Beta-Carotin ungeeignet. Die reinen Ölpräparate gehen von der Injektionsstelle nur langsam in den Blutstrom über und die zwischenzeitlich zwangsläufig an der Einstichstelle erfolgende Deposition von Öl und Beta-Carotin ist für das Tier mit erheblichen Schmerzen und histopathologischen Veränderungen verbunden.

Emulsionen sind insoferne problematisch, als sie nur eine geringe Stabilität gegenüber spontan eintretender Phasentrennung besitzen. Überdies ist die Beständigkeit von Beta-Carotin gegen Oxidation durch den in der Luft enthaltenen Sauerstoff, in Emulsionen weiter verschlechtert.

Die DE 196 09 477 A beschreibt wässerige Solubilisate von Carotinoiden, darunter Beta-Carotin, und Vitaminen, die mit Hilfe von nichtionogenen Emulgatoren, z.B. Polyoxyethylen-12-hydroxystearat mit 10 bis 40 Oxyethyleneinheiten, in einer Konzentration von 1 bis 40 Gew.-% hergestellt sind. Dabei kann der Gehalt an Carotinoiden in den aus der DE 196 09 477 A bekannten Solubilisaten 0,1 bis 10 Gew.-% betragen.

Aus EP 0 055 817 A sind Beta-Carotin-Solubilisate bekannt, die als Stabilisator ein nicht-ionogenes Tensid, beispielsweise ethoxylierte Triglyceride, enthalten.

In der FR 2 187 291 A ist Isopropylmyristat als nicht-ionogener Emulgator für Beta-Carotin beschrieben.

Der Erfindung liegt daher die Aufgabe zugrunde, eine zur parenteralen Verabreichung geeignete, wässerige Zubereitung von Beta-Carotin und ein Verfahren zum Herstellen derselben zur Verfügung zu stellen.

Gelöst wird diese Aufgabe bei einer Zubereitung mit den Merkmalen des Anspruches 1 und was das Verfahren anlangt, mit einer Arbeitsweise nach Anspruch 8.

Ein wesentliches Merkmal der Erfindung ist es, daß Beta-Carotin in der erfindungsgemäßen Zubereitung als mizellare Lösung (Mikroemulsion) vorliegt, so daß bei lichtgeschützter Lagerung bei normaler Raumtemperatur eine uneingeschränkte Verwendungsfähigkeit der Zubereitung über einen Zeitraum von mindestens zwei Jahren gewährleistet ist, sowohl was den Gehalt an Beta-Carotin, als auch was die Stabilität der Zubereitung hinsichtlich Phasentrennung betrifft.

Die zur parenteralen Verabreichung, insbesondere zur intramuskulären Injektion, geeignete Zubereitung gemäß der Erfindung, die beispielsweise Huf- und Klauentieren, sowie Hunden verabreicht werden kann, weist einen Gehalt an Beta-Carotin von 0,1% bis 10% (w/v), insbesondere 1 bis 5% (w/v), auf. Die erfindungsgemäße mizellare Lösung von Beta-Carotin in dem wässerigen Medium enthält ein Gemisch von Isopropylmyristat und Polyoxyethylen-660-hydroxystearat , wobei die Konzentration von Isopropylmyristat 5 bis 20% (w/v) und die Konzentration von Polyoxyethylen-660-hydroxystearat 10 bis 40% (w/v) beträgt. Bevorzugte Konzentrationen sind 5 bis 10% (w/v) für Isopropylmyristat und 15 bis 20% (w/v) für Polyoxyethylen-660-hydroxystearat.

Die erfindungsgemäße Zubereitung enthält wenigstens ein Antioxidans. Geeignete Antioxidantien sind Ascorbylpalmitat und/oder DL-alpha-Tocopherol enthalten. Der Gehalt an Antioxidans kann 0,01 bis 0,1% (w/v), bevorzugt 0,02 bis 0,03% (w/v) betragen.

Ein Verfahren zum Herstellen der erfindungsgemäßen mizellaren Lösung von Beta-Carotin in einem zur parenteralen Verabreichung geeigneten wässerigen Medium wird nachstehend anhand des angeschlossenen Fließbildes eines beispielhaften und bevorzugten Ablaufes eines Herstellungsverfahrens erläutert. Die im Fließbild verwendete Abkürzung "IPK" bedeutet "In-Prozeß-Kontrolle".

Im ersten Verfahrensschritt werden Polyoxyethylen-660-hydroxystearat und Isopropylmyristat in der Menge eingewogen, daß die Konzentration von Isopropylmyristat in der fertigen Zubereitung 5 bis 20% (w/v) und die Konzentration von Polyoxyethylen-660-hydroxystearat in der fertigen Zubereitung 10 bis 40% (w/v) beträgt. Dieses Gemisch wird unter Rühren bis zum Vorliegen einer klaren Lösung erwärmt.

Die so erhaltene klare Lösung wird unter Rühren mit der einem Gehalt an Beta-Carotin in der fertigen Zubereitung von 0,1 bis 10% (w/v) entsprechenden Menge an Beta-Carotin versetzt, wobei beim Auflösen des Beta-Carotins eine Temperatur von 100 bis 140°C (vorzugsweise 118 bis 128°C) eingehalten wird. Es wird so lange gerührt, bis eine dunkelrote, klare Lösung erhalten worden ist.

Die so erhaltene Lösung wird auf 75°C ± 2°C abgekühlt. Nun werden als Antioxidantien Ascorbylpalmitat und DL-alpha-Tocopherol zugegeben, wobei solche Mengen zugegeben werden, daß jedes der Antioxidantien in einer Menge von 0,005 bis 0,05% (w/v) enthalten ist.

Die so erhaltene Mischung wird unter Rühren portionsweise mit Wasser für Injektionszwecke versetzt und nach dem Abkühlen auf 30°C als Konservierungsmittel Benzylalkohol zugegeben. Nach der Zugabe von Benzylalkohol (in einer Menge von 10 mg/ml) wird die restliche Menge an Wasser zur Injektion bei Raumtemperatur zugesetzt und dann das Aussehen, der pH-Wert und die Dichte der so erhaltenen mizellaren Lösung (Mikroemulsion) geprüft.

Die so erhaltene fertige Lösung wird sterilfiltriert.

Nach der Sterilfiltration wird aseptisch abgefüllt, die Glasflaschen konfektioniert, verpackt und eine Endkontrolle hinsichtlich Aussehen, Füllvolumen, Identität- und Gehaltbestimmung der Wirkstoffe und Sterilität ausgeführt.

Das so erhaltene Präparat stellt eine zur parenteralen Verabreichung geeignete Zubereitung von Beta-Carotin in einem wässerigen Medium dar, die bei lichtgeschützter Lagerung bei Raumtemperatur über einen Zeitraum von zwei Jahren verwendbar bleibt.

Die Abkürzung "% (w/v)" bezieht sich auf die Masse des jeweiligen Stoffes in Prozent bezogen auf das Volumen der fertigen, wässerigen Zubereitung von Beta-Carotin.

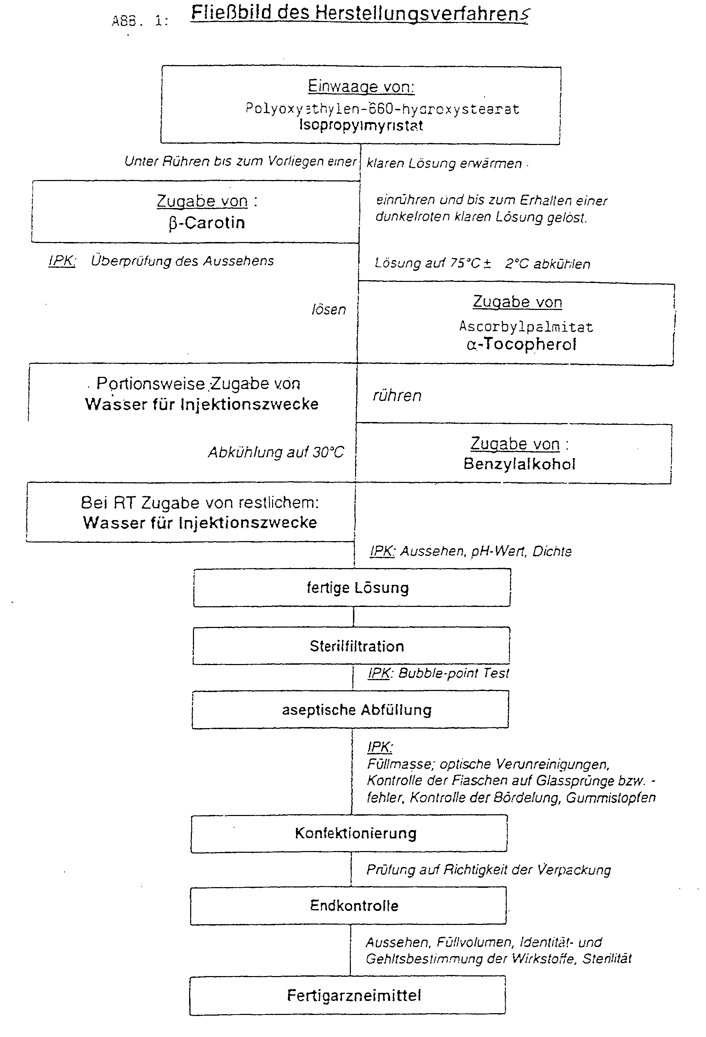

## Patentansprüche

1. Zubereitung von Beta-Carotin in einem wässerigen Medium zur parenteralen Verabreichung, in Form einer mizellaren Lösung, die Lösungsvermittler enthält, **dadurch gekennzeichnet, daß** der Gehalt an Beta-Carotin 0,1 bis 10%. (w/v) beträgt, daß die Zubereitung als Lösungsvermittler Polyoxyethylen-660-hydroxystearat und Isopropylmyristat enthält, daß die Konzentration von Polyoxyethylen-660-hydroxystearat in der Zubereitung 10 bis 40% (w/v), daß die Konzentration von Isopropylmyristat in der Zubereitung 5 bis 20% (w/v) beträgt und daß die Zubereitung wenigstens ein Antioxidans enthält.

2. Zubereitung nach Anspruch 1, **dadurch gekennzeichnet, daß** die Konzentration von Polyoxyethylen-660-hydroxystearat in der Zubereitung 15 bis 20% (w/v) beträgt.

3. Zubereitung nach Anspruch 1 oder 2, **dadurch gekennzeichnet, daß** der Gehalt an Beta-Carotin in der Zubereitung 1 bis 5% (w/v) beträgt.

4. Zubereitung nach Anspruch 1, **dadurch gekennzeichnet, daß** die Konzentration von Isopropylmyristat in der Zubereitung 5 bis 10% (w/v), beträgt.

5. Zubereitung nach Anspruch 1, **dadurch gekennzeichnet, daß** das Antioxidans aus der Gruppe bestehend aus Ascorbylpalmitat und DL-alpha-Tocopherol und Kombinationen derselben ausgewählt ist.

6. Zubereitung nach Anspruch 5, **dadurch gekennzeichnet, daß** die Konzentration an Antioxidans 0,01 bis 0,1% (w/v), insbesondere 0,02 bis 0,03% (w/v) beträgt.

7. Zubereitung nach Anspruch 5, **dadurch gekennzeichnet, daß** die Konzentration von Ascorbylpalmitat und DL-alpha-Tocopherol jeweils 0,005 bis 0,05% (w/v) beträgt.

8. Verfahren zum Herstellen einer Zubereitung nach einem der Ansprüche 1 bis 7, **dadurch gekennzeichnet, daß** man in eine auf 70°C bis 140°C erwärmte Lösung von Polyoxyethylen-660-hydroxystearat und Isopropylmyristat, vorzugsweise unter Rühren, Beta-Carotin einträgt, daß man in die so erhaltene Polyoxyethylen-660-hydroxystearat, Isopropylmyristat und Beta-Carotin enthaltende, auf 75°C ± 2°C erwärmte Lösung wenigstens ein Antioxidans einträgt, und daß man die Polyoxyethylen-660-hydroxystearat, Isopropylmyristat und Beta-Carotin und wenigstens ein Antioxidans enthaltende Lösung portionsweise mit Wasser zur Injektion verdünnt.

9. Verfahren nach Anspruch 8, **dadurch gekennzeichnet, daß** man als Antioxidans Ascorbylpalmitat und/oder Alpha-Tocopherol einträgt.

10. Verfahren nach Anspruch 8 oder 9, **dadurch gekennzeichnet, daß** man die mit Wasser zur Injektion verdünnte Lösung von Polyoxyethylen-660-hydroxystearat, Isopropylmyristat, Beta-Carotin und wenigstens einem Antioxidans nach dem Abkühlen auf 30°C ± 5°C mit Konservierungsmittel, insbesondere Benzylalkohol, vorzugsweise in einer Menge von 10 mg/ml der Lösung, versetzt.

## Claims

1. Preparation of beta-carotene in an aqueous medium for parenteral administration, in the form of a micellar solution, which contains solutiser, **characterised in that** the beta-carotene content amounts to 0.1 to 10% (w/v), that the preparation contains polyoxyethylene-660-hydroxy stearate and isopropyl myristate as solutiser, that the concentration of polyoxyethylene-660-hydroxy stearate in the preparation amounts to 10 to 40% (w/v), that the concentration of isopropyl myristate in the preparation amounts to 5 to 20% (w/v) and that the preparation contains at least one antioxidant.

2. Preparation according to Claim 1, **characterised in that** the concentration of polyoxyethylene-660-hydroxy stearate in the preparation amounts to 15 to 20% (w/v).

3. Preparation according to Claim 1 or 2, **characterised in that** the content of beta-carotene in the preparation amounts to 1 to 5% (w/v).

4. Preparation according to claim 1, **characterised in that** the concentration of isopropyl myristate in the preparation amounts to 5 to 10% (w/v).

5. Preparation according to Claim 1, **characterised in that** the antioxidant is selected from the group comprising ascorbyl palmitate and DL-alpha-tocopherol and combinations thereof.

6. Preparation according to Claim 5, **characterised in that** the concentration of antioxidant amounts to 0.01 to 0.1% (w/v), in particular 0.02 to 0.03% (w/v).

7. Preparation according to Claim 5, **characterised in that** the concentration of ascorbyl palmitate and DL-alpha-tocopherol respectively amounts to 0.005 to 0.05% (w/v).

8. Method for the production of a preparation according to one of Claims 1 to 7, **characterised in that** beta-carotene is incorporated into a solution heated to 70°C to 140°C of polyoxyethylene-660-hydroxy stearate and isopropyl myristate, preferably while stirred, that at least one antioxidant is incorporated into the resulting solution heated to 75°C ± 2°C containing polyoxyethylene-660-hydroxy stearate, isopropyl myristate and beta-carotene, and that the solution containing polyoxyethylene-660-hydroxy stearate, isopropyl myristate and beta-carotene and at least one antioxidant is diluted with water in portions for injection.

9. Method according to Claim 8, **characterised in that** ascorbyl palmitate and/or alpha-tocopherol is incorporated as antioxidant.

10. Method according to Claim 8 or 9, **characterised in that** after cooling to 30°C ± 5°C, the solution of polyoxyethylene-660-hydroxy stearate, isopropyl myristate, beta-carotene and at least one antioxidant diluted with water for injection is mixed with preservative, in particular benzyl alcohol, preferably in a quantity of 10 mg/ml of solution.

## Revendications

1. Préparation de bêta-carotène dans un milieu aqueux pour l'administration parentérale sous forme d'une solution micellaire contenant un agent de solubilisation, **caractérisée en ce que** la teneur en bêta-carotène est comprise entre 0,1 et 10 % poids/volume, que la préparation contient comme agent de solubilisation du polyoxyéthylène-660-hydroxystéarate et du myristate d'isopropyle, que la concentration de polyoxyéthylène-660-hydroxystéarate dans la préparation est comprise entre 10 et 40 % poids/volume, que la concentration de myristate d'isopropyle dans la préparation est comprise entre 5 et 20 % poids/volume et que la préparation contient au moins un antioxydant .

2. Préparation suivant la revendication 1, **caractérisée en ce que** la concentration de polyoxyéthylène-660-hydroxystéarate dans la préparation est comprise entre 15 et 20 % poids/volume.

3. Préparation suivant la revendication 1 ou 2, **caractérisée en ce que** la teneur de bêta-carotène dans la préparation est comprise entre 1 et 5 % poids/volume.

4. Préparation suivant la revendication 1, **caractérisée en ce que** la concentration de myristate d'isopropyle dans la préparation est comprise entre 5 et 10 % (m/v).

5. Préparation suivant la revendication 1, **caractérisée en ce que** l'antioxydant est choisi parmi le groupe composé d'ascorbylpalmitate et de DL-alpha-tocophérol et de combinaisons de ceux-ci.

6. Préparation suivant la revendication 5, **caractérisée en ce que** la concentration en antioxydants est comprise entre 0,01 et 0,1 % poids/volume, en particulier entre 0,02 et 0,03 %.

7. Préparation suivant la revendication 5, **caractérisée en ce que** la concentration d'ascorbylpalmitate et de Dl-alpha-tocophérol est respectivement comprise entre 0,005 et 0,05% poids/volume.

8. Procédé de fabrication d'une préparation suivant une des revendications 1 à 7, **caractérisé en ce que** l'on ajoute du bêta-carotène à une solution de polyoxyéthylène-660-hydroxystéarate et de myristate d'isopropyle chauffée à une température comprise entre 70°C et 140°C, de préférence en agitant, et que l'on ajoute au moins un antioxydant à la solution contenant du polyoxyéthylène-660-hydroxystéarate, du myristate d'isopropyle et du bêta-carotène et chauffée à 75°C ± 2°C, et que la solution contenant du polyoxyéthylène-660-hydroxystéarate, du myristate d'isopropyle et du bêta-carotène est diluée, par portions, dans de l'eau pour I'injection.

9. Procédé suivant la revendication 8, **caractérisé en ce que** l'on ajoute comme antioxydant de l'ascorbylpalmitate et /ou de l'alpha-tocophérol.

10. Procédé suivant la revendication 8 ou 9, **caractérisé en ce que** l'on ajoute à la solution de polyoxyéthylène-660-hydroxystéarate, de myristate d'isopropyle, de bêta-carotène et d'un antioxydant au moins, diluée dans de l'eau pour l'injection, un agent conservateur, en particulier de l'alcool benzylique, de préférence une quantité de 10 mg/ml de la solution, une fois la solution refroidie à 30°C ± 5°C.
